# EUROPEAN PATENT SPECIFICATION

(11) **EP 4 364 691 B1**
(45) Date of publication and mention of the grant of the patent: **09.12.2026**
(21) Application number: 22833482.7
(22) Date of filing: 21.06.2022
(51) Int. Cl.: A61C 8/00, A61L 27/34, A61L 27/50, A61L 27/36

(54) **IMPLANT FIXTURE FOR REDUCING PERIODONTITIS**
IMPLANTATHALTERUNG ZUR REDUZIERUNG VON PERIODONTITIS
ÉLÉMENT DE FIXATION D'IMPLANT POUR RÉDUIRE LA PARODONTITE

(30) Priority: 02.07.2021 KR 20210087373
(43) Date of publication of application: 08.05.2024
(73) Proprietor: Hong Sung Inc., Seoul 04512 (KR)
(72) Inventor: LEE, Eui Seok, Seoul 06288 (KR)
(74) Representative: Tetzner, Michael
(86) International application number: PCT/KR2022/008749
(87) International publication number: WO 2023/277426

(56) References cited:
- WO-A1-00/72775
- WO-A1-2018/030662
- KR-A- 20150 000 670
- KR-B1- 100 414 885
- KR-B1- 100 983 919
- KR-B1- 101 122 134
- KR-B1- 101 325 246
- KR-B1- 101 909 690
- US-A- 5 989 027
- US-A1- 2017 340 416
- US-A1- 2021 153 982

## Description

### Technical Field

The present disclosure relates to an implant fixture for reducing periodontitis.

### Background Art

In general, when permanent teeth are damaged due to aging or other factors, artificial teeth are implanted into the jawbone. When permanent teeth are damaged due to various factors and the damaged permanent teeth are extracted, artificial teeth are implanted in their place.

Specifically, prosthetic implant components are attached to implant fixtures implanted in the jawbone, and artificial teeth are combined with the implant to complete the implantation of the artificial teeth. Typical implants used for implanting artificial teeth consist of a fixture, an abutment, and a screw. The fixture is inserted into the jawbone and coupled by threads, and, in a state in which the abutment to which the artificial teeth will be fixed is located above the fixture, the screw is connected to the abutment, and then, is coupled to the fixture, such that the abutment is coupled to the top of the fixture. Generally, the fixture has threads formed along the outer surface thereof, such that the fixture is coupled to the jawbone.

However, if the implant does not fully integrate with the alveolar bone, the connection of the implant fixture along the thread may become loose from the gums. If load is continuously applied in a state in which a portion of the implant fixture is disassembled, a gap may be formed, and the load transferred by the implant fixture is concentrated on a portion of the bone tissue, so the bone tissue is damaged and the implant fixture is detached from the bone tissue.

Additionally, conventional methods simply use the shape of the implant and the surface treatment technique for bone integration. However, the currently used implant systems have several disadvantages in that, after implantation of the implant, peri-implantitis is likely to occur, the rate of bone integration survival is often low due to the small surface area of the implant, and frequent fractures of the implant itself occur due to the application of occlusal force.

To overcome the limitations of traditional implants, the surface area of the implant fixture must be increased to increase the success rate of bone integration and survival rate. In particular, it is necessary to develop an implant system that can increase the survival rate by reducing the occurrence of peri-implantitis.

The background technology of the present invention is disclosed in Korean Patent No. 10-1122134. US 5989027 A describes a dental implant for osseointegration in alveolar bone, the implant including an annular shoulder and a plurality of splines extending axially therefrom for interdigitated engagement with similar splines of an abutment for supporting a tooth prosthesis, wherein the bone-engaging surface of the implant includes a biocompatible porous metal surface into which bone can grow, an intermediate non-porous, rough, biocompatible metal surface to which bone can attach that is located between the porous surface and the annular shoulder, and a smooth, non-porous biocompatible metal surface located between the intermediate surface and the annular shoulder. WO 0072775 A1 describes an implant for application in bone (8), comprising a unit which can be applied in the bone in question and which is made of biocompatible material, wherein on its surface parts cooperating with the bone, the unit is provided with a coating of an agent TS, which initiates and/or stimulates bone growth, wherein the coating comprises calcium phosphate compounds CaP and the said stimulating agent TS.

US2021153982A1 shows a monolithic dental implant made by additive manufacturing and milling, it comprises a non-porous head portion with a tapered collar at a coronal end of the implant and a porous middle portion positioned below the non-porous head portion, the non-porous head portion comprises micro threading and the porous middle portion comprises macro threading.

### Disclosure

### Technical Problem

Accordingly, the present invention has been made in view of the above-mentioned problems occurring in the related art, and it is an object of the present invention to provide an implant fixture for reducing periodontitis which can widen the surface area of the outer surface to increase fixing force to the bone tissue, and provide bone formation inducers to at least a portion of the outer surface to increase stability and the success rate of bone integration.

However, the technical problems that the embodiments of the present invention aim to solve are not limited to the aforementioned technical problems, and there may exist other technical problems to be solved.

### Technical Solution

The present invention provides an implant fixture according to claim 1. Preferred embodiments of the invention are defined in the dependent claims. To accomplish the above-mentioned objects, according to the present invention, there is provided an implant fixture for reducing periodontitis including: a body part which is implanted into bone tissue to form an artificial tooth root, wherein the outer surface of the body part has a predetermined roughness to enhance fixing force of the body part to the bone tissue, and at least a portion of the outer surface of the body part is provided with bone formation inducers, wherein an upper portion of the body part has a machined surface with smoother surface characteristics compared to the outer surface of the body part to improve adhesion with soft tissue, wherein, the body part includes: a first body part corresponding to a predetermined depth section downwards from the machined surface; and a second body part corresponding to the lower end of the fixture,
wherein the first body part is provided in a fin-type including a plurality of wing members, and the second body part is provided in a screw-type with threads formed on the outer surface thereof so that the first body part and the second body part have distinct outer surface characteristics, and the wing members are made of a material with higher strength compared to the material of the second body part

Additionally, the bone formation inducer provided on the outer surface of the first body part is a first type inducer, and the bone formation inducer provided on the outer surface of the second body part is a second type inducer, different from the first type inducers.

In addition, the first type inducer includes poly lactic-co glycolic acid (PLGA).

Moreover, the second type inducer includes bone morphogenetic protein (BMP).

Furthermore, the implant fixture for reducing periodontitis according to an embodiment of the present invention may further include a cavity formed by recessing upwards from the lower end of the body part.

Additionally, after the implant fixture is implanted, at least a portion of the bone tissue can be drawn into the cavity.

In addition, the implant fixture for reducing periodontitis according to an embodiment of the present invention may include at least one through-hole formed to allow communication between the cavity and the outside of the body part.

Moreover, the bone formation inducers are provided on the inner surface of the cavity.

Furthermore, the bone formation inducers are provided at a plurality of different positions on the outer surface, each at a predetermined distance from each other.

Additionally, a plurality of dimple-shaped concave grooves are formed on the outer surface of the body part.

In addition, the bone formation inducers are provided corresponding to the plurality of grooves.

In addition to the illustrative embodiments mentioned above, additional embodiments may exist in the drawings and detailed description of the present invention.

### Advantageous Effect

According to the present invention, the implant fixture for reducing periodontitis can widen the surface area of the outer surface to increase fixing force to the bone tissue, and provide the bone formation inducers to at least a portion of the outer surface to increase stability and the success rate of bone integration.

According to an embodiment of the present invention, the implant fixture for reducing periodontitis includes the cavity recessed upwards from the lower portion of the body such that a portion of the bone tissue is drawn inwards after the implant is placed, thereby preventing fracture of the implant and significantly improving the fixation of the implant.

However, the effects obtainable from the present invention are not limited to those mentioned above, and other effects may exist.

### Description of Drawings

FIG. 1 is a schematic diagram of an embodiment of an implant fixture for reducing periodontitis according to the present invention.
FIG. 2 is a conceptual diagram for explaining the positioning and spacing of bone formation inducers provided on the outer surface of a body part.
FIG. 3 is a perspective view of the implant fixture including a cavity according to a first embodiment of the present invention.
FIG. 4 is a longitudinal sectional view of the implant fixture including the cavity according to the first embodiment of the present invention.
FIG. 5 is a perspective view of the implant fixture including a cavity according to a second embodiment of the present invention.
FIG. 6 is a longitudinal sectional view of the implant fixture including the cavity according to the second embodiment of the present invention.
FIG. 7 is a perspective view of the implant fixture including a cavity according to a third embodiment of the present invention.
FIG. 8 is a longitudinal sectional view of the implant fixture including the cavity according to the third embodiment of the present invention.
FIG. 9 is a perspective view of the implant fixture including a cavity according to a fourth embodiment of the present invention.
FIG. 10 is a longitudinal sectional view of the implant fixture including the cavity according to the forth embodiment of the present invention.

### Mode for Invention

Hereinafter, the embodiments of the present disclosure will be described in detail with reference to accompanying drawings so that the embodiments may be easily implemented by those skilled in the art. However, the present disclosure may be implemented in various ways without being limited to the embodiments. In addition, in the drawings, well-known elements or components may be omitted to avoid unnecessarily obscuring the presented embodiments, and like reference numerals denote like elements throughout the specification.

In the entire specification of the present disclosure, when a part is "connected" to another part, this includes not only being "directly connected" but also being "electrically connected" or "indirectly connected" through another element.

In the entire specification of the present disclosure, when any member is located "on," "above," "at the top of," "below," "beneath," or "at the bottom of" another member, this includes a case in which still another member is prevent between both members as well as a case in which one member is in contact with another member.

In the entire specification of the present disclosure, when any portion "includes" any component, this does not exclude other components but means that any other component can be further included, unless stated otherwise.

The present invention relates to an implant fixture for reducing periodontitis.

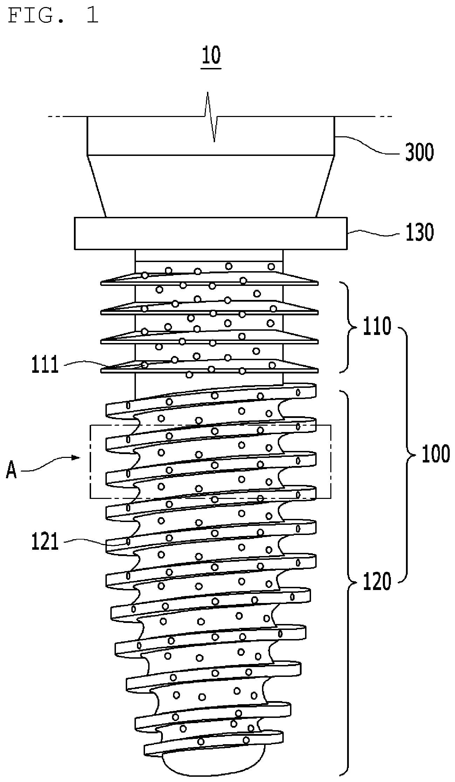

FIG. 1 is a schematic diagram of the implant fixture for reducing periodontitis according to an embodiment of the present invention.

Referring to FIG. 1, the implant fixture 10 for reducing periodontitis (hereinafter, referred to as 'implant fixture 10') which illustrates the present invention includes a body part 100 which is to be implanted into bone tissue to form an artificial tooth root.

Moreover, the outer surface of the body part 100 is provided with a predetermined roughness to enhance fixing force of the body part 100 to the bone tissue.

Furthermore, an upper portion of the body part 100 has a machined surface 130 with smoother surface characteristics compared to the outer surface of the body part 100 to improve adhesion with soft tissue.

In other words, the implant fixture 10 disclosed in the present invention is configured such that the machined surface 130 with a smooth surface is formed on the top of the implant fixture to prevent damage to the gum tissue where the implant is placed and to improve adhesion with the soft tissue area. In this instance, the body part 100 below the machined surface 130 has a rougher surface than the machined surface 130 to enhance the fixing force to the bone tissue in the area where the implant is placed.

Meanwhile, in the present invention, the body part 100 is divided into a first body part 110 and a second body part 120 based on a predetermined depth section in the vertical direction.

The first body part 110 corresponds to a preset depth section downwards from the bottom surface of the machined surface 130, and the second body part 120 corresponds to the lower end of the implant fixture 10 that lies beneath the first body part 110.

In this regard, the outer surface characteristics of the first body part 110 and the second body part 120 are distinct from each other. The first body part 110, which is a relatively upper area, is provided in a fin-type, and the second body part 120, which is a relatively lower area, is provided in a screw-type with threads formed on the outer surface thereof.

In this regard, the shape of the upper first body part 110, which is positioned corresponding to the boundary between the soft tissue and the bone tissue of the site where the implant fixture 10 is implanted, is provided in a fin-type including a plurality of wing members. The wing members are made of a material with higher strength compared to the material of the second body part 120, which is located relatively below, thereby reducing the risk of fracture of the implant fixture 10.

In other words, according to the present invention, the strength of the first body part 110 is set higher than that of the second body part 120, thereby reducing the risk of fracture of the implant fixture 10.

Moreover, according to the present invention, at least a portion of the outer surface of the body part 100 is provided with bone formation inducers (111, 121) .

Specifically, the bone formation inducers (111) provided on the outer surface of the first body part 110 may be a first type inducers. Distinctly, the bone formation inducers (121) provided on the outer surface of the second body part 120 may be a second type inducers, different from the first type inducers (111) of the first body part 110.

According to an embodiment of the present invention, the first type inducer may include poly lactic-co glycolic acid (PLGA). Additionally, according to an embodiment of the present invention, the second type inducer may include bone morphogenetic protein (BMP).

Although, out of conventionally developed implant fixture structures, some of implant fixture structures applying bone integration inducing substances like bone morphogenetic protein (BMP) on the entire outer surface have been disclosed. However, in the type where the bone formation (bone integration) inducing substances are applied to the entire outer surface of the fixture, despite requiring a relatively long period of time for stable bone integration after the implantation of the implant fixture, as most of the bone formation inducing substances applied to the entire outer surface of the fixture are absorbed into the bone tissue at the initial stage right after the implantation of the implant, so such conventional implant fixture structures often fail to achieve the effect of promoting bone integration by the bone formation inducing substances.

In contrast, the implant fixture 10 disclosed in the present invention provides the bone formation inducers on the outer surface of the body part 100. For example, the bone formation inducers are arranged locally at intervals in some areas of the outer surface, so that the bone formation inducers are provided (released) gradually to the bone tissue over a relatively long period of time, thereby enabling effective bone integration over an extended duration.

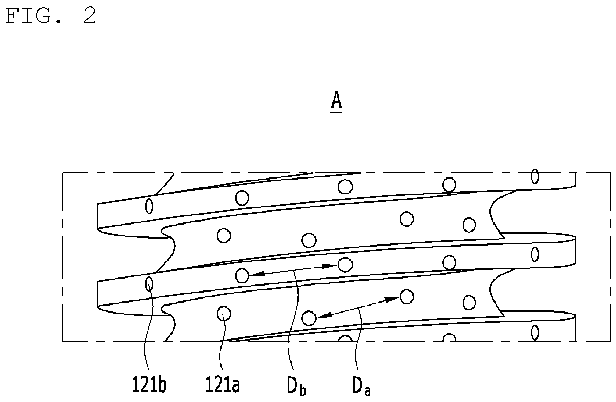

FIG. 2 is a conceptual diagram to explain the positioning and spacing of the bone formation inducers provided on the outer surface of the body part.

Referring to FIG. 2, the bone formation inducers may be provided at multiple different locations on the outer surface of the body part 100, each location having a predetermined distance from each other.

According to an embodiment of the present invention, the screw type second body part 120 is provided with a protrusion area protruding toward the outside with the threads and a recessed area recessed inwards between the threads, the arrangement and spacing between areas to which each of the bone formation inducers is provided can be applied differently, so that an appropriately sustained release of the bone formation inducer towards the bone tissue can be achieved.

More specifically, referring to FIG. 2, assuming that the spacing distance of the bone formation inducers 121a placed in the recessed area of the second body part 120 is given as Da, and the spacing distance of the bone formation inducers 121b placed in the protrusion area is given as Db, Da can be set larger than Db.

In addition, referring to FIG. 2, the bone formation inducers 121a placed in the recessed area can be alternately provided (arranged), based on the vertical direction, in a relatively upward area and a relatively downward area respectively in the circumferential direction of the outer surface of the second body part 120, providing the bone formation inducers to the bone tissue more uniformly.

According to an embodiment of the present invention, a plurality of dimple-shaped concave grooves (not shown) are formed on the outer surface of the body part 100, and the bone formation inducers may be provided corresponding to the plurality of grooves.

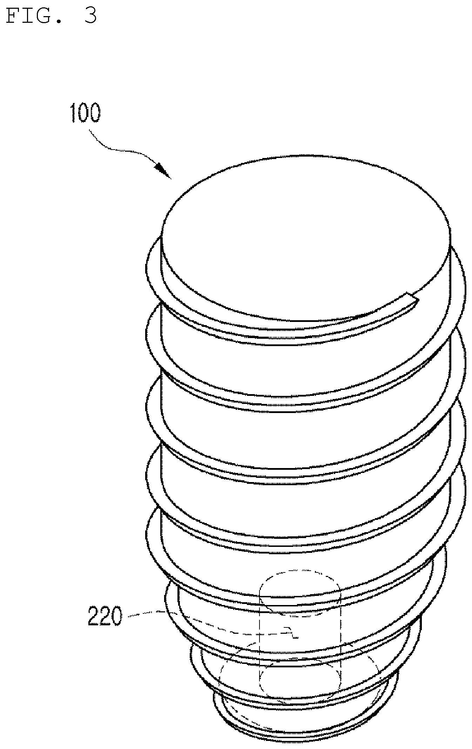

Hereinafter, referring to FIGS. 3 to 10, a cavity 220 formed at the lower end of the body part 100 and a through hole 230 that allows communication between the cavity 220 and the outside of the body part 100 will be described to improve the fixing force of the implant fixture 10.

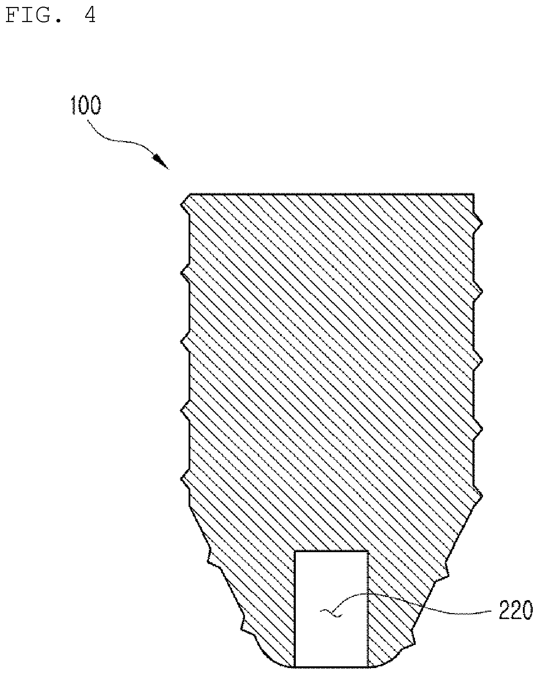

FIG. 3 is a perspective view of the implant fixture including the cavity according to a first embodiment of the present invention, and FIG. 4 is a longitudinal sectional view of the implant fixture including the cavity according to the first embodiment of the present invention.

Referring to FIGS. 3 and 4, the cavity 220 can be formed by recessing upwards from the lower end of the body part 100. In this regard, after the implantation of the fixture 10, at least a portion of the bone tissue can be drawn into the cavity 220.

According to the first embodiment of the present invention, the cavity 220 can be formed as a space surrounded by the lower interior of the body part 100, i.e., at the lower end of the body part 100.

Accordingly, once the body part 100 is implanted into the bone tissue, at least a portion of the bone tissue enters and settles inside the cavity 220, and then, is hardened, such that the body part 100 is more strongly fixed, thereby enhancing the fixing force and durability of the implant fixture 10.

Additionally, according to an embodiment of the present invention, the inner surface of the cavity 220 may be provided with bone formation inducers, similar to the outer surface of the body part 100. Furthermore, according to an embodiment of the present invention, the bone formation inducers provided on the inner surface of the cavity 220 can be provided to the bone tissue that is at least partially drawn into the cavity 220 after a predetermined period of time following the implantation of the implant fixture 10, thereby continuously promoting the improvement of the fixing force of the implant by inducing bone integration through the bone formation inducers over a long period.

In addition, according to an embodiment of the present invention, considering that it takes a predetermined period of time for at least some of the bone tissue to be drawn into the cavity 220, the bone formation inducers provided on the inner surface of the cavity 220 may be provided at a relatively higher area ratio compared to those provided on the outer surface of the body part 100 that bonds with the bone tissue right after the implantation of the implant fixture 10.

For example, the size (such as diameter) of the dimple-shaped concave grooves (not shown) formed on the inner surface of the cavity 220 to provide the bone formation inducers can be set larger than those of the plurality of grooves (not shown) formed on the outer surface of the body part 100, or the spacing distance between multiple positions where the bone formation inducers are provided may be smaller than the outer surface of the body part 100.

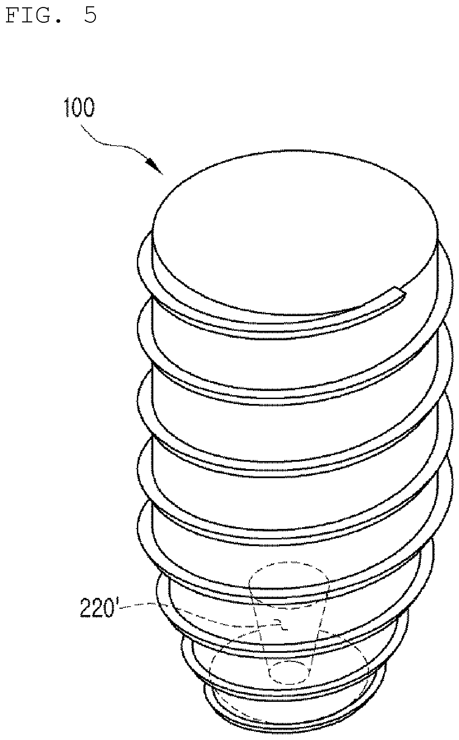

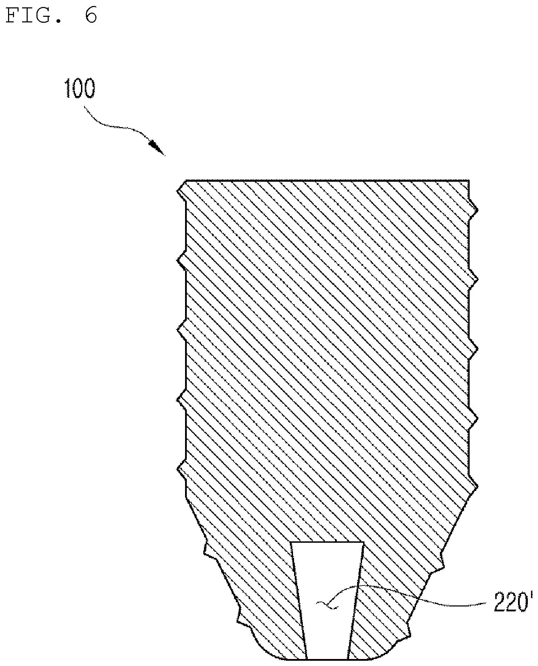

FIG. 5 is a perspective view of the implant fixture including a cavity according to a second embodiment of the present invention, and FIG. 6 is a longitudinal sectional view of the implant fixture including the cavity according to the second embodiment of the present invention.

Referring to FIGS. 5 and 6, the implant fixture 10 according to the second embodiment of the present invention can have a cavity 220 with a tapered shape decreasing in diameter from the top to the bottom. Consequently, when the body part 100 is implanted, at least a portion of the bone tissue entering and hardening inside the cavity 220 not only strengthens the fixation of the body part 100 but also prevents the body part 100 from being pulled out from the implantation site due to the aforementioned taper shape.

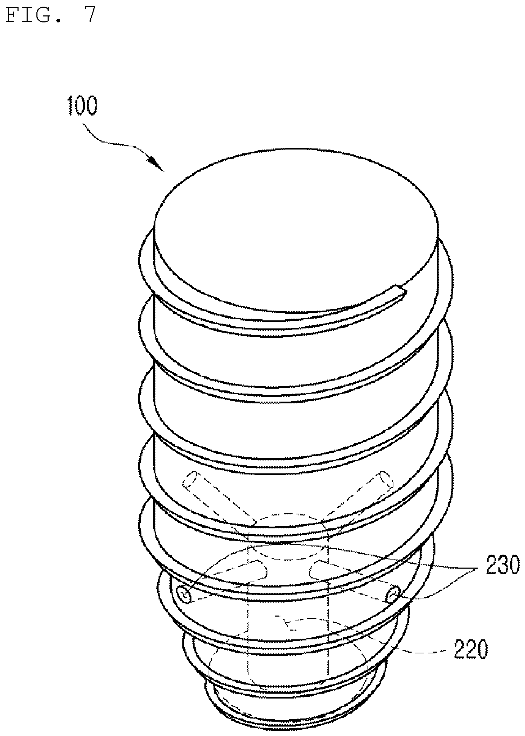

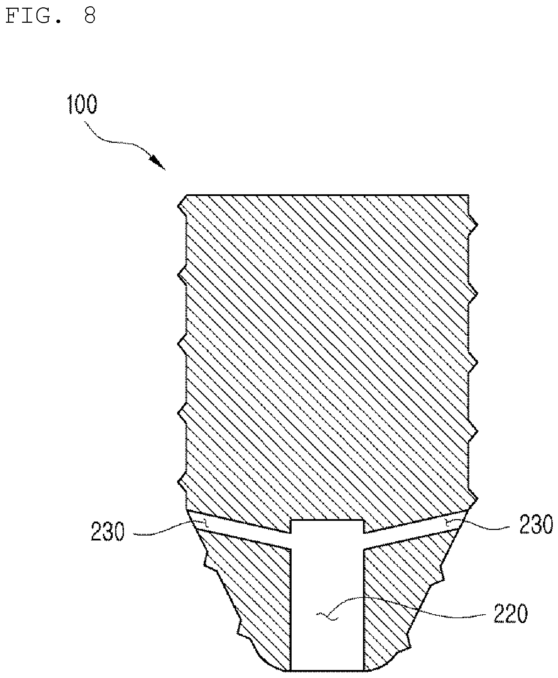

FIG. 7 is a perspective view of the implant fixture including a cavity according to the third embodiment of the present invention, and FIG. 8 is a longitudinal sectional view of the implant fixture including a cavity according to the third embodiment of the present invention.

Referring to FIGS. 7 and 8, the implant fixture 10 according to the third embodiment of the present invention may have at least one through-hole 230 formed to allow communication between the cavity 220 and the outside of the body part 100.

According to this third embodiment, in case of the implant fixture 10 with the through-hole 230, the bone formation inducers provided on the inner surface of the cavity 220 pass through the through-hole 230 after the implantation of the implant fixture 10, and are provided to the bone tissue located outside the outer surface of the body part 100, thereby continuously promoting the enhancement of the fixing force of the implant by inducing bone integration through the bone formation inducers over a long period.

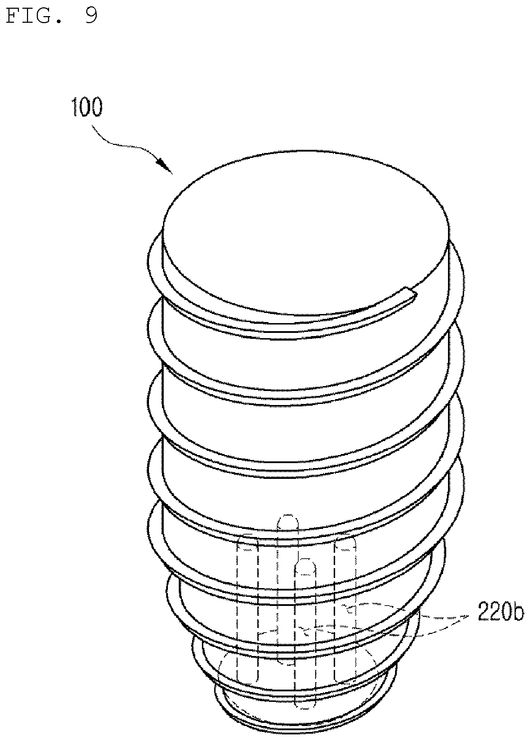

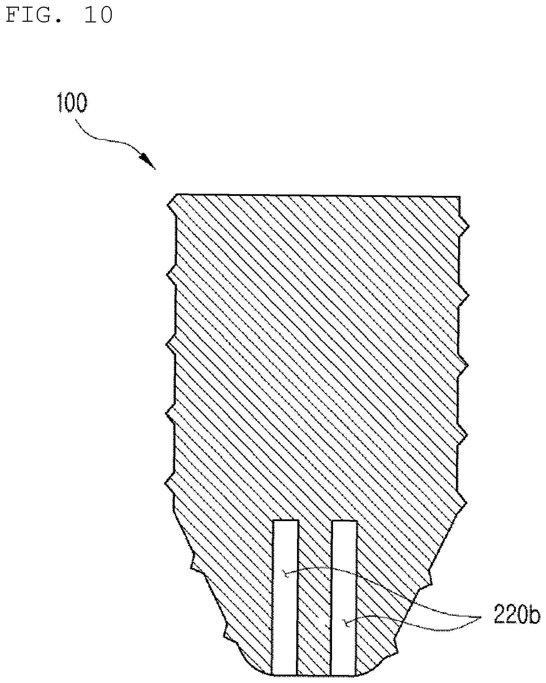

FIG. 9 is a perspective view of the implant fixture including a cavity according to a fourth embodiment of the present invention, and FIG. 10 is a longitudinal sectional view of the implant fixture including a cavity according to the fourth embodiment of the present invention.

Referring to FIGS. 9 and 10, the implant fixture 10 according to the fourth embodiment of the present invention may have a plurality of cavities 220b, which are spaces surrounded by the lower end of the body part 100. As a result, when the body part 100 is inserted into the implantation site, at least a portion of the bone tissue enters and settles inside the plurality of cavities 220b, thereby providing enhanced fixing force of the body part 100.

The above description is only exemplary, and it will be understood by those skilled in the art that the invention may be embodied in other concrete forms , the scope of the invention being defined by the appended claims Therefore, some above-described embodiments should be considered only as examples in all aspects and not for purposes of limitation. For example, each component described as a single type may be realized in a distributed manner, and similarly, components that are described as being distributed may be realized in a coupled manner.

## Claims

1. An implant fixture for reducing periodontitis comprising:
a body part (100) which is implanted into bone tissue to form an artificial tooth root,
wherein the outer surface of the body part (100) has a predetermined roughness to enhance fixing force of the body part (100) to the bone tissue, and at least a portion of the outer surface of the body part (100) is provided with bone formation inducers,
wherein an upper portion of the body part (100) has a machined surface (130) with smoother surface characteristics compared to the outer surface of the body part (100) to improve adhesion with soft tissue,
wherein the body part (100) comprises:
a first body part (110) corresponding to a predetermined depth section downwards from the machined surface (130); and
a second body part (120) corresponding to the lower end of the fixture (10),
wherein the first body part(110) is provided in a fin-type including a plurality of wing members, and the second body part(120) is provided in a screw-type with threads formed on the outer surface thereof so that the first body part(110) and the second body part(120) have distinct outer surface characteristics, and the wing members are made of a material with higher strength compared to the material of the second body part (120).

2. The implant fixture according to claim 1, wherein the bone formation inducer provided on the outer surface of the first body part (110) is a first type inducer, and
wherein the bone formation inducer provided on the outer surface of the second body part (120) is a second type inducer, different from the first type inducers.

3. The implant fixture according to claim 2, wherein the first type inducer includes poly lactic-co glycolic acid (PLGA), and the second type inducer includes bone morphogenetic protein (BMP).

4. The implant fixture according to claim 1, further comprising:
a cavity (220) formed by recessing upwards from the lower end of the body part (100),
wherein after the implant fixture (10) is implanted, at least a portion of the bone tissue is drawn into the cavity (220).

5. The implant fixture according to claim 4, wherein at least one through-hole (230) is formed to allow communication between the cavity (220) and the outside of the body part (100).

6. The implant fixture according to claim 4, wherein bone formation inducers are provided on the inner surface of the cavity (220).

7. The implant fixture according to claim 1, wherein the bone formation inducers are provided at a plurality of different positions on the outer surface, each at a predetermined distance from each other.

8. The implant fixture according to claim 7, wherein a plurality of dimple-shaped concave grooves are formed on the outer surface of the body part (100), and
wherein the bone formation inducers are provided corresponding to the plurality of grooves.

## Patentansprüche

1. Implantatkörper zur Verringerung einer Parodontitis, umfassend: eine Körperteil (100), der in Knochengewebe implantiert wird, um einen künstlichen Zahnwurzelersatz zu bilden, wobei die Außenfläche des Körperteils (100) eine vorbestimmte Rauheit aufweist, um die Haltekraft des Körperteils (100) am Knochengewebe zu erhöhen, und wobei mindestens ein Abschnitt der Außenfläche des Körperteils (100) mit Knochenbildungsinduktoren versehen ist,
wobei ein oberer Abschnitt des Körperteils (100) eine bearbeitete Oberfläche (130) mit glatteren Oberflächeneigenschaften als die Außenfläche des Körperteils (100) aufweist, um die Haftung am Weichgewebe zu verbessern,
wobei das Körperteil (100) umfasst:
einen ersten Körperabschnitt (110), der einer vorbestimmten Tiefe unterhalb der bearbeiteten Oberfläche (130) entspricht; und
einen zweiten Körperabschnitt (120), der dem unteren Ende des Implantatkörpers (10) entspricht, wobei der erste Körperabschnitt (110) lamellenförmig mit mehreren Flügelelementen ausgebildet ist und der zweite Körperabschnitt (120) schraubenförmig mit Gewinde auf seiner Außenfläche ausgebildet ist, sodass der erste Körperabschnitt (110) und der zweite Körperabschnitt (120) unterschiedliche Außenflächeneigenschaften aufweisen, und wobei die Flügelelemente aus einem Material mit höherer Festigkeit als das Material des zweiten Körperabschnitts (120) bestehen.

2. Implantatkörper nach Anspruch 1, wobei der an der Außenfläche des ersten Körperabschnitts (110) vorgesehene Knochenbildungsinduktor ein Induktor erster Art ist, und wobei der an der Außenfläche des zweiten Körperabschnitts (120) vorgesehene Knochenbildungsinduktor ein Induktor zweiter Art ist, der sich von den Induktoren erster Art unterscheidet.

3. Implantatkörper nach Anspruch 2, wobei der Induktor erster Art Polymilchsäure-co-Glykolsäure (PLGA) und der Induktor zweiter Art ein knochenmorphogenetisches Protein (BMP) umfasst.

4. Implantatkörper nach Anspruch 1, ferner umfassend: einen Hohlraum (220), der durch Ausnehmung von dem unteren Ende des Körperteils (100) nach oben ausgebildet ist, wobei nach dem Implantieren des Implantatkörpers (10) mindestens ein Abschnitt des Knochengewebes in den Hohlraum (220) eingezogen wird.

5. Implantatkörper nach Anspruch 4, wobei mindestens eine Durchgangsbohrung (230) ausgebildet ist, die eine Verbindung zwischen dem Hohlraum (220) und der Außenseite des Körperteils (100) herstellt.

6. Implantatkörper nach Anspruch 4, wobei Knochenbildungsinduktoren an der Innenfläche des Hohlraums (220) vorgesehen sind.

7. Implantatkörper nach Anspruch 1, wobei die Knochenbildungsinduktoren an mehreren verschiedenen Positionen der Außenfläche vorgesehen sind, wobei jeweils ein vorbestimmter Abstand zwischen ihnen besteht.

8. Implantatkörper nach Anspruch 7, wobei an der Außenfläche des Körperteils (100) mehrere grubenförmige konkave Rillen ausgebildet sind und wobei die Knochenbildungsinduktoren entsprechend den mehreren Rillen vorgesehen sind.

## Revendications

1. Fixation d'implant pour réduire la parodontite, ladite fixation d'implant comprenant :
une partie (100) formant le corps, qui est implantée dans le tissu osseux pour former une racine dentaire artificielle,
fixation d'implant dans laquelle la surface externe de la partie (100) formant le corps présente une rugosité prédéterminée afin d'augmenter la force de fixation de la partie (100) formant le corps au tissu osseux, et au moins une partie de la surface externe de la partie (100) formant le corps est dotée d'inducteurs de formation osseuse,
fixation d'implant dans laquelle une partie supérieure de la partie (100) formant le corps a une surface usinée (130) présentant des caractéristiques de surface plus lisses par comparaison avec la surface externe de la partie (100) formant le corps, afin d'améliorer l'adhérence aux tissus mous,
fixation d'implant dans laquelle la partie (100) formant le corps comprend :
une première partie (110) formant le corps, qui correspond à une section de profondeur prédéterminée et orientée vers le bas à partir de la surface usinée (130) ; et
une seconde partie (120) formant le corps, qui correspond à l'extrémité inférieure de la fixation (10), fixation d'implant dans laquelle la première partie (110) formant le corps est du type à ailette comprenant une pluralité de segments d'ailes, et la seconde partie (120) formant le corps est du type à vis comprenant des filetages formés sur sa surface externe, de sorte que la première partie (110) formant le corps et la seconde partie (120) formant le corps ont des caractéristiques de surface externe distinctes, et les segments d'ailes sont constitués d'un matériau ayant une résistance supérieure comparée à celle du matériau de la seconde partie (120) formant le corps.

2. Fixation d'implant selon la revendication 1, dans laquelle l'inducteur de formation osseuse, qui est prévu sur la surface externe de la première partie (110) formant le corps, est un inducteur de premier type, et fixation d'implant dans laquelle l'inducteur de formation osseuse, qui est prévu sur la surface externe de la seconde partie (120) formant le corps, est un inducteur de second type différent des inducteurs de premier type.

3. Fixation d'implant selon la revendication 2, dans laquelle l'inducteur de premier type comprend de l'acide polylactique-co-glycolique (PLGA), et l'inducteur de second type comprend de la protéine morphogénétique osseuse (BMP).

4. Fixation d'implant selon la revendication 1, comprenant en outre :
une cavité (220) formée par un évidement orienté vers le haut à partir de l'extrémité inférieure de la partie (100) formant le corps,
où, après la mise en place de la fixation d'implant (10), au moins une partie du tissu osseux est aspirée dans la cavité (220).

5. Fixation d'implant selon la revendication 4, dans laquelle au moins un trou traversant (230) est formé pour permettre la communication entre la cavité (220) et l'extérieur de la partie (100) formant le corps.

6. Fixation d'implant selon la revendication 4, dans laquelle des inducteurs de formation osseuse sont prévus sur la surface interne de la cavité (220).

7. Fixation d'implant selon la revendication 1, dans laquelle les inducteurs de formation osseuse sont prévus au niveau d'une pluralité de positions différentes sur la surface externe, chacune d'elles étant à une distance prédéterminée les unes par rapport aux autres.

8. Fixation d'implant selon la revendication 7, dans laquelle une pluralité de rainures concaves en forme de fossette sont formées sur la surface externe de la partie (100) formant le corps, et
fixation d'implant dans laquelle les inducteurs de formation osseuse sont prévus de façon correspondant à la pluralité de rainures.
